Europäisches Patentamt

European Patent Office

Office européen des brevets

(11) Publication number: **0 103 201**
**B1**

## (12) EUROPEAN PATENT SPECIFICATION

(45) Date of publication of patent specification: 30.05.90

(51) Int. Cl.⁵: **C 12 N 15/00**

(21) Application number: **83108090.8**

(22) Date of filing: **16.08.83**

(54) **Shuttle vector.**

(30) Priority: 20.08.82 JP 145093/82
16.08.82 JP 142460/82

(43) Date of publication of application:
21.03.84 Bulletin 84/12

(45) Publication of the grant of the patent:
30.05.90 Bulletin 90/22

(84) Designated Contracting States:
AT BE CH DE FR GB IT LI NL SE

(56) References cited:
EP-A-0 100 561

PROCEEDINGS OF THE NATIONAL ACADEMY
OF SCIENCES USA, vol. 79, April 1982, pages
2157-2161, Washington, US; D.T. ROGERS et
al.: "Acid phosphatase polypeptides in
Saccharomyces cerevisiae are encoded by a
differentially regulated multigene family"

(73) Proprietor: Juridical Foundation The Chemo-
Sero-Therapeutic Research Institute
668, Okubo Shimizu-machi
Kumamoto-shi Kumamoto-ken (JP)

(72) Inventor: Miyanohara, Atsushi
8-16, Kori-motomachi Neyagawa-shi
Osaka-fu (JP)
Inventor: Toh-e, Akio
2-2-1, Mitaki-hommachi Nishi-ku
Hiroshima-shi Hiroshima-ken (JP)
Inventor: Matsubara, Kenichi
3-1-57-909, Fukushima Fukushima-ku
Osaka-shi Osaka-fu (JP)

(74) Representative: Vossius & Partner
Siebertstrasse 4 P.O. Box 86 07 67
D-8000 München 86 (DE)

(56) References cited:

PROCEEDINGS OF THE NATIONAL ACADEMY
OF SCIENCES USA, vol. 80, January 1983, pages
1-5, Washington, US; A. MIYANOHARA et al.:
"Expression of hepatitis B surface antigen gene
in yeast"

## Description

The present invention relates to a novel shuttle vector. More particularly it relates to a shuttle vector which contains saccharomyces cerevisiae derived DNA sequences and DNA sequences of the *Escherichia coli* plasmid pBR322 and the expression control region of the repressible acid phosphatase gene of yeast said region being hereinafter referred to as "acid phosphatase promoter" or "acid phosphatase gene"). This shuttle vector can replicate both in *E. coli* and in yeast.

With recent advances in genetic engineering, various recombinant DNAs and transformants prepared therefrom have been developed. In the preparation of the recombinant DNAs, a vector for inserting a specific gene is used. Such a vector includes a vector which can replicate only in a certain microorganism, e.g. in *Escherichia coli*, and a so-called shuttle vector which can replicate in two or more kinds of microorganisms, e.g. both in *E. coli* and in a yeast, or both in a certain microorganism (e.g. *E. coli*) and in a certain animal cell. For instance, a shuttle vector is known which can replicate both in *E. coli* and in yeast, i.e. a vector utilizing the alcohol dehydrogenase (ADH1) expression control region to achieve downstream promotion of the HBsAg structural gene (cf. P. Valenzuela et al., Nature, *298*, 347—350; 22 July, 1982).

If a DNA sequence coding for HBsAg is inserted into this vector and the recombinant plasmid is used for preparing a transformant, the transformant can produce the desired HBsAg only in a small amount.

The technical problem underlying the present invention is to provide an improved *E. coli*-yeast shuttle vector in which various DNA sequences encoding polypeptides can be inserted and can be expressed in yeast. This problem is solved by a shuttle vector which comprises the following components:

(a) DNA sequences of the yeast *Saccharomyces cerevisiae* including ars 1, the 2 μori, and a marker gene for a transformed yeast permitting the synthesis of leucine, histidine, tryptophane, uracil or adenine by the transformant;

(b) a DNA sequence of the *E. coli* plasmid pBR 322 which includes a marker gene for a transformed *E. coli* encoding a resistance for ampicillin as well as the origin of replication; and

(c) the expression control region of the repressible acid phosphatase gene of yeast without the entire phosphatase structural gene and without a region in the range of from +1 (ATG) to −100 bp upstream of the structural gene.

The shuttle vector used in the present invention is a plasmid vector which contains both DNA sequences of the yeast *Saccharomyces cerevisiae* a DNA sequence of the *E. coli* plasmid pBR 322 and the expression control region and the repressible acid phosphatase gene of yeast. This plasmid vector can replicate both in *E. coli* and in yeast, and can be used for the preparation of recombinant plasmids which in turn are used for the preparation of transformed yeast. The transformed yeast thus prepared can produce the desired gene products in a large scale.

The yeast derived DNA sequence usually contains a DNA sequence which is necessary for the replication of a plasmid in the yeast as an episome, for instance, a DNA sequence necessary for the autonomous replication in yeast (which is designated "ars 1"), and a DNA sequence necessary for the replication of 2 μm DNA (which is designated "2 μ ori"), and contains a gene useful as a selective marker for the transformed yeast. The selective marker includes a leucine-producing gene, a histidine-producing gene, a tryptophane-producing gene, a uracil-producing gene or an adenine-producing gene, which may be used alone or in combination of two or more thereof.

The pBR 322 derived dNA sequences contain a DNA sequence necessary for the replication of the plasmid in *E. coli*, for example a DNA sequence of a replication initiating region of the plasmid Col El and a marker gene encoding ampicillin resistance for the transformed *E. coli*.

The shuttle vector of the present invention is characteristic in that it carries the repressible acid phosphatase promoter of yeast. This acid phosphatase promoter is usually a promoter controlling the expression of a polypeptide of 60,000 daltons (p60) which constitutes the acid phosphatase.

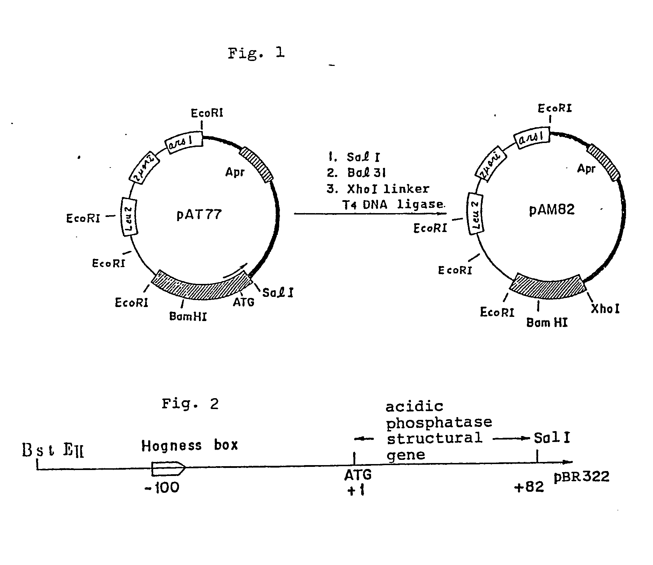

A representative example of the shuttle vector is a vector which is prepared by recombining a yeast derived DNA containing the ars 1, 2 μ ori and a leucine producing gene (leu 2) with the *E. coli* plasmid pBR322. The shuttle vector is designated "pAT 77" (Fig. 1) and is prepared as follows.

An EcoRI fragment of about 8,000 base pairs (8 kb) containing a DNA sequence coding for a polypeptide (p60) of 60,000 daltons which constitutes the repressible acid phosphatase (cf. PNAS, *77*, 6541—6545, 1980, and PNAS, *79*, 2157—2161, 1982) is inserted into the EcoRI site of the *E. coli* plasmid pBR322 (cf. Sutcliffe, J. G.; Cold Spring Harbor Symposium, *43*, 77—90, 1979) to give a plasmid, which is used as the starting material. Said EcoRI fragment (8 kb DNA fragment) contains a unique recognition site for the restriction enzyme Sal I at a position where it is cleared into a fragment of about 2.8 kb and a fragment of about 5.2 kb.

The starting plasmid is digested with the restriction enzyme Sal I and re-annealed with T4 DNA ligase to give a plasmid which has lost the 5.2 kb acid phosphatase gene fragment. This plasmid is designated "pAT 25". pAT 25 is a plasmid consisting of a 3.7 kb fragment extending from the EcoRI site to the Sal I site of pBR322 which contains the ampicillin-resistance gene and a fragment (about 2.8 kb) extending from the EcoRI site to the Sal I site of the yeast acid phosphatase gene.

An EcoRI fragment (1.4 kb) containing a DNA sequence necessary for the autonomous replication of the ars 1 and trp 1 gene of yeast (cf. PNAS, *76*, 1035—1039, 1979) is inserted into the EcoRI site of pAT 25 in order to obtain plasmid "pAT 26". Said ars 1-trp 1 fragment contains a unique recognition site for the restriction enzyme Hind III within the trp 1 gene.

A Hind III fragment containing the leu 2 gene of yeast and a DNA sequence necessary for the replication of 2 μm DNA (2 μ ori) is inserted into this unique Hind III site of the recombinant plasmid pAT 26 (cf. Tohe, A. et al., J. Bacteriol., *141*, 413—416, 1980) in order to obtain the desired shuttle vector pAT 77.

The plasmid pAT 77 and pAM 82 derived therefrom as described hereinafter have the structures as shown in Figure 1. In Figure 1, the dark line represents the EcoRI/Sal I—pBR322-fragment containing the ampicillin-resistance gene (Apr) linked to ars 1, 2 μ ori, and the leu 2 gene in this order and to the 2.8 kb EcoRI/Sal I—fragment of the acid phosphatase gene. The recombinant plasmid pAT 77 can replicate in *E. coli* because of the presence of the origin of replication of pBR322 and it can also replicate in yeast because of the presence of the ars 1 and 2 μ ori genes. Moreover, this plasmid contains as markers in order to select for transformed *E. coli* the ampicillin-resistance gene (Apr) and for transformed yeast the leu 2 gene, and thereby fulfills the requirements to be used as a shuttle vector.

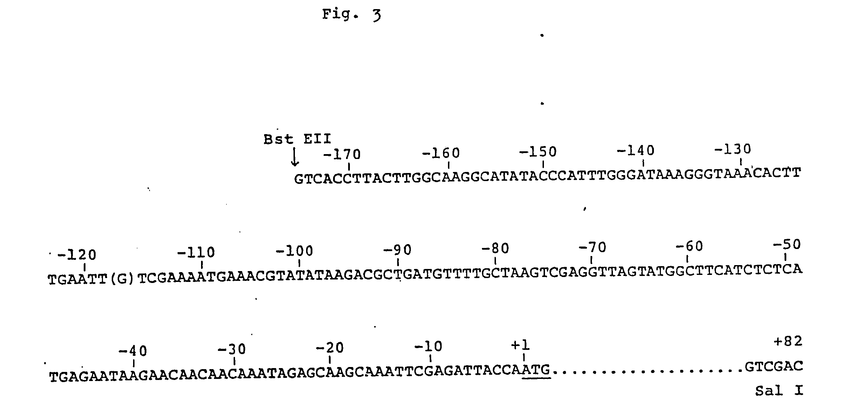

The gene map around the acid phosphatase promoter of the shuttle vector pAT 77 is shown in Figure 2. The nucleotide sequence of the BstEII/Sal I region in this shuttle vector is shown in Figure 3. The ATG codon (methionine) shown in Figures 2 and 3 is the start codon of the acid phosphatase structural gene. That is, the repressible about 2.8 kb acid phosphatase gene fragment of this vector contains the region of from about 2.7 kb upstream from the structural gene to the 82nd nucleotide base pair (82 bp) of the structural gene.

The shuttle vector pAT 77 is cleaved with the restriction enzyme Sal I, followed by treating with the exonuclease Bal 31, by which a part or the complete structural gene of the acid phosphatase as shown in Figures 2 and 3 and further optionally various regions upstream therefrom are deleted. This deletion is effected for appropriate regions before the acid phosphatase promoter region: TATATAA (Hogness box), i.e. −100 bp. The regions to be deleted can be controlled by the conditions for treating with the exonuclease Bal 31 and are usually in the range of from +1 to −100 bp, preferably from +1 to −50 bp and most preferably from +1 to −33 bp. The loss of the Hogness-box by extensive deletion, i.e. over −100 bp implies the loss of the control region, resulting in a lower yield of the desired gene products in the cultivated transformed yeast cells. On the other hand, when the deletion is insufficiently effected so that a part of the acid phosphatase structural gene remains, a fusion protein of a foreign gene product and a phosphatase peptide may be formed which is not desirable.

After deleting the complete structural gene of the acid phosphatase and some regions upstream thereof, a synthetic or natural linker, for example a Sal I linker or Xho I linker, is recombined thereto to give a circular plasmid. This recombinant plasmid is a shuttle vector in which a foreign gene can be inserted the expression of which is controlled by the acid phosphatase promoter. This shuttle vector can readily be cleaved at a site to be recombined by treating it with a conventional restriction enzyme, such as Sal I or Xho I, and hence, is preferably used in order to recombine with the desired gene.

The shuttle vector of the present invention can be used for the preparation of various recombinant plasmids by recombining it with various genes downstream of the acid phosphatase promoter and further for the preparation of various transformed yeasts with the recombinant plasmids. Hence, it is very useful in the field of genetic engineering. For instance, the shuttle vector of the present invention is used for the preparation of a recombinant plasmid comprising a DNA sequence coding for a Hepatitis B virus (HBV) surface antigen (HBsAg). The recombinant plasmid is used for preparing a transformed yeast which can produce HBsAg in a large scale. The HBsAg obtained behaves like the natural HBs antigen from human donors in terms of immunological properties and hence is useful for the preparation of Hepatitis B virus vaccine.

The present invention is illustrated by the following Example and Preparation.

Example

Preparation of the shuttle vectors pAM 81, 82, 83 and 84

An EcoRI fragment of about 8,000 base pairs (8 kb) containing a DNA sequence coding for a polypeptide (p60) of 60,000 daltons which constitutes the repressible acid phosphatase (available from Yeast S288C gene bank, Clarke, L. and Carbon, J., Cell, *9*, 91—99, 1976) is inserted into the EcoRI site of the *E. coli* plasmid pBR322 to give a plasmid, which is used as the starting material.

The starting plasmid is digested with the restriction enzyme Sal I and re-annealed with T4 DNA ligase to give plasmid pAT25 which has lost the 5.2 kb acid phosphatase gene fragment plasmid pAT 25 is a plasmid consisting of a 3.7 kb fragment extending from the EcoRI site to the Sal I site of pBR322 which contains the ampicillin-resistance gene and a 2.8 kb fragment extending from the EcoRI site to the Sal I site of the yeast acid phosphatase gene.

An EcoRI fragment (1.4 kb) containing the ars 1 and trp 1 gene which is prepared by treating a plasmid YRP 7 (cf. Struhl, K. et al., Proc. Natl. Acad. Sci. U.S.A., *76*, 1035—1039, 1979) with EcoRI is inserted into the EcoRI site of pAT 25 in order to obtain plasmid pAT 26. Said ars 1-trp 1 fragment contains a unique recognition site for the restriction enzyme Hind III within the trp 1 gene.

A Hind III fragment containing a leu 2 and 2 μ ori which is prepared by treating the plasmid pSLE 1 (cf. Tohe, A. et al., J. Bacteriol., *141*, 413—416, 1980) with Hind III is inserted into the Hind III site of pAT 26 in order to obtain the desired shuttle vector pAT 77. *Saccharomyces cerevisiae* harbouring pAT 77 (i.e.

*Saccharomyces cerevisiae* AH 22/pAT 77) has been deposited at the Fermentation Research Institute, Agency of Industrial Science and Technology, Japan according to the Budapest Treaty as "FERM BP-324".

The pAT 77 thus obtained (1 µg) is cleaved with Sal I and is then treated with the exonuclease Bal 31 (0.1 U) in a solution (50 µl) of 20 mM Tris-HCl (pH 8.2), 12 mM CaCl$_2$, 12 mM MgCl$_2$, 0.2 M NaCl and 1 mM EDTA for 30 seconds to one minute. The reaction mixture is subjected to phenol extraction and ethanol precipitation in the same manner as described in Preparation (1) (ii) (A) hereinafter. The resulting precipitates are treated with an Xho I linker (1 pmol) and T4 DNA ligase under the same conditions as described in Preparation (1) (ii) (A) hereinafter for 12 hours.

*E. coli* x1776 is treated with the above reaction mixture by the procedure as described in R. Curtiss III et al., "Molecular cloning of recombinant DNA", eds. W. A. Scott and R. Werner, page 99, Academic Press (1977), so as to transform the *E. coli* x1776 to give an ampicillin-resistant transformant. From the resulting transformant colonies, plasmid DNAs are prepared by the procedure as described by K. Matsubara (J. Virol., *16*, 479, 1975). According to the Maxam-Gilbert method (cf. Maxam, A. & Gilbert, W.; P.N.A.S., *74*, 560—564, 1977), the nucleotide sequence of the resulting DNAs is determined. Furthermore the region of the acid phosphatase gene deleted with Bal 31 is determined. Among these DNAs, the desired plasmids pAM 81, pAM 82, pAM 83 and pAM 84 which are deficient in the complete structural gene of phosphatase are selected and isolated.

Designating "A" in the codon ATG encoding the first amino acid (methionine) of the product p60 of the phosphatase structural gene as "+1", the following regions are deleted in these shuttle vectors, pAM 81: till +2, pAM 82: till −33, pAM 83: till −50, and pAM 84: till −51. *Saccharomyces cerevisiae* harbouring pAM 81, pAM 82, pAM 83 and pAM 84 (i.e. *Saccharomyces cerevisiae* AH 22/pAM 81, AH 22/pAM 82, AH 22/pAM 83 and AH 22/pAM 84, respectively) have been deposited at the Fermentation Research Institute, Agency of Industrial Science and Technology, Japan according to the Budapest Treaty as "FERM BP-325", "FERM BP-313", "FERM BP-327", and "FERM BP-326", respectively.

An embodiment of preparing a recombinant plasmid, a transformed yeast and HBsAg by recombination of HBV DNA using the shtutle vector pAM 82 is illustrated in the following Preparation.

Preparation
(1) Preparation of HBV DNA
(i) Preparation of virus DNA

Pooled blood plasma (700 ml) obtained from ten patients who are positive in HBsAg (subtype adr) and HBcAg is centrifuged at 5,000 r.p.m. for 20 minutes to remove undissolved substances. The resulting solution is centrifuged at 4°C, 18,000 r.p.m. for 8 hours, and the resultant precipitate is re-dissolved in 10 ml of a buffer (pH 7.5) of 10 mM Tris-HCl, 0.1 M NaCl and 1 mM EDTA. The solution is added to the top of a centrifuge tube containing 30% sucrose, which is centrifuged at 4°C, 39,000 r.p.m. for 4 hours. The resultant precipitate is redissolved in the same buffer as above.

In order to allow the following operations the buffer solution is subjected to the reaction with HBV DNA polymerase by treating it in a mixture (500 µl) of 67 mM Tris-HCl (pH 7.5), 80 mM NH$_4$Cl, 25 mM MgCl$_2$, 0.5% NP40 (Tergitol®, manufactured by Sigma Co.), 0.1%. 2-Mercaptoethanol, 330 µM dCTP (deoxycytidine triphosphate), dGTP (deoxyguanosine triphosphate), and dATP (deoxyadenosine triphosphate), 0.5 µM α-[$^{32}$P]dTTP (deoxythymidine triphosphate), at 37°C for 3 hours. To the reaction mixture is added the same volume of 100 mM EDTA solution. By the above DNA polymerase reaction, the single-stranded region of the DNA is repaired to a completely double-stranded structure which is [$^{32}$P] labelled. This DNA is added to the top of a centrifuge tube wherein 30%, 20% and 10% aqueous solutions of sucrose are packed in layers in this order, and it is centrifuged at 4°C, 39,000 r.p.m. for 4.5 hours.

In order to digest the proteins strongly bonded to DNA, the precipitates obtained above are treated in a mixture (200 µl) of 1 mg/ml of Pronase E (manufactured by Kaken Kagaku K.K.) and 0.2% aqueous sodium lauryl sulfate solution at 37°C for 2 hours. The resulting mixture is extracted with phenol (200 µl) twice, and the resulting DNA-containing extract is washed with ether to remove the phenol and to give a solution of HBV DNA. The DNA thus obtained has a specific radioactivity of 2.5×10$^6$ cpm/µg and can be used for digestion with restriction enzymes.

(ii) Cloning of HBV DNA

The double-stranded circular HBV DNA obtained above is cloned by using λ-phage Charon 16A DNA as a vector and then it is subcloned by using the known plasmid pACYC177 as a vector as follows.

(A) Cloning in the system of λ-phage Charon 16A host-vector:

HBV DNA (20 ng) is treated with endonuclease Xho I in a mixture (20 µl) of 10 mM Tris-HCl (pH 7.4), 7 mM MgCl$_2$, 100 mM NaCl and 7 mM 2-mercaptoethanol at 37°C for 2 hours. The resulting mixture is extracted with phenol (20 µl) and further with ether, and to the aqueous layer is added a double volume of cooled ethanol to precipitate DNA. The mixture is kept at −70°C for one hour and then centrifuged at 10,000 r.p.m. for 5 minutes, and the precipitated DNA is recovered. The precipitate thus separated is dissolved in a mixture (5 µl) of 10 mM Tris-HCl (pH 7.4) and 1 mM EDTA. The HBV DNA and an equimolar amount of λ-phage Charon 16 A DNA (having a unique Xho I recognition site) obtained by cleavage with endonuclease Xho I in the same manner as described above are reacted with T4 DNA ligase [a mixture of 50 mM Tris-HCl

4

(pH 7.4), 10 mM MgCl$_2$, 10 mM dithiothreitol, 100 µg/ml calf serum albumin, 0.5 mM ATP and 0.5 µl enzyme preparation (T4 ligase, manufactured by Takara Biomedicals, 1—5×10$^3$ unit/ml)] at 4°C for 18 hours. The reaction mixture is extracted with phenol and ether and then subjected to precipitation with ethanol in the same manner as described above. The precipitates thus obtained are dissolved in a mixture (10 µl) of 10 mM Tris-HCl (pH 7.4) and 1 mM EDTA.

The thus annealed DNA is subjected to in vitro packaging operation to form λ-phages in the same manner as described in "Methods in Enzymology", *68*, 299—309. Furthermore plaques (10$^4$) are formed therefrom on an L-agar plate (23 cm×23 cm) by using *E. coli* DP50 SupF (cf. Blattner, F. R. et al., Science *196*, 161, 1977) as an indicator. These plaques are subjected to plaque hybridization using $^{32}$P-labelled HBV DNA prepared above as a probe (cf. Science, *196*, 180, 1977) in order to select plaques formed from the phages having HBV DNA. A plurality of the desired phages are separated.

(B) Subcloning by using plasmid pACYC177 as a vector:

From HBV DNA cloned in Charon 16 A as obtained in step (A) a phage DNA is prepared by using *E. coli* DP50-SupF as bacteria to be infected in the same manner as described in "Methods in Enzymology", *68*, 245—378, 1979. The DNA thus obtained is digested with Xho I under the same conditions as described above for 2 hours, and the resulting reaction mixture is subjected to an electrophoresis on a 0.75% agarose gel to isolate HBV DNA (3.2 kb). The HBV DNA is absorbed onto DEAE (diethylaminoethyl cellulose) paper (manufactured by Toyo Roshi, Japan) in order to separate it from the vector DNA and then eluted with 1 M NaCl aqueous solution to give an HBV DNA having Xho I termini at both ends.

Separately, plasmid pACYC177 (cf. Chang, A. C. Y., Cohen, S. N.; J. Bacteriol., *134*, 1141—1156, 1978) having a unique Xho I cleavage site within its kanamycin-resistance gene is digested with Xho I, and the product is purified by phenol extraction, ether treatment and ethanol precipitation in the same manner as described above.

The thus obtained pACYC177 cleaved with Xho I is mixed with XhoI-terminus HBV DNA obtained above in a molar ratio of 1:5, and the mixture is annealed and ligated with T4 DNA ligase for 18 hours as described above.

The annealed DNA preparation (10 µl) obtained above is added to a suspension of *E. coli* (0. 1 ml) which is prepared by treating a culture broth of *E. coli* x1776 [cf. R. Curtiss III et al., "Molecular cloning of recombinant DNA" eds. W. A. Scott and R. Werner, page 99, Academie Press (1977)] by the procedure as described in M. V. Norgard, Gene, *3*, 279 (1978). The mixture is mixed well and allowed to stand at 0°C for 25 minutes. The mixture is applied onto an L-agar plate containing ampicillin (20 µg/ml), α-biotine (1 µg/ml), diaminopimelic acid (100 µg/ml) and thymine (20 µg/ml) and is incubated at 37°C overnight. The resulting colonies are applied onto both an agar plate containing kanamycin (20 µg/ml) and an agar plate containing ampicillin (20 µg/ml). The colonies which grow only on the agar plate containing ampicillin are selected. pACYC177 contains an ampicillin-resistance gene and a kanamycin-resistance gene, but when the HBV DNA is inserted into the Xho I site of the kanamycin-resistance gene, the kanamycin-resistance is lost. Accordingly, the selected colonies contain a recombinant DNA of pACYC177-HBV DNA. From the colonies thus selected, a plasmid is prepared by the procedure as described by K. Matsubara (J. Virol., *16*, 479, 1975). The plasmid thus obtained, i.e. the recombinant DNA of pACYC177-HBV DNA (which is designated "pHBV"), is treated with Xho I under the same conditions as described above to give a complete HBV DNA fragment (3.2 kb). Besides, when it is treated with Xho I and BamHI, there is obtained a fragment (about 1.3 kb) containing the HBsAg gene.

(2) Preparation of HBsAg expression plasmids

(i) Preparation of plasmids containing the complete HBV DNA

HBV DNA obtained by treating plasmid pHBV (pACYC177-HBV DNA) with Xho I is recombined with the Xho I cleaved shuttle vector, pAM 81, pAM 82, pAM 83 or pAM 84 in a molar ratio of 5:1 by annealing and ligating with T4 DNA ligase under the same conditions as described above.

*E. coli* x1776 is transformed with the reaction mixture and plasmid DNA is prepared from the resulting ampicillin-resistant transformant in the same manner as described hereinbefore. The DNAs thus prepared are analyzed with various restriction enzymes, such as Xho I, Xba I and Hind III, and thereby the insertion of HBV DNA into the vectors and its orientation are determined.

The thus obtained HBsAg gene-expression plasmids contain the HBs and HBc coding sequence in this order downstream of the phosphatase promoter, and the plasmid is designated pAH 203.

(ii) Preparation of the plasmid containing the HBsAg coding sequence

An HBsAg coding sequence (3 µg) prepared by cleaving plasmid pHBV with BamHI is treated with T4 DNA polymerase (0.2 U) in a solution (100 µl) of 67 mM Tris-HCl (pH 8.6), 6.7 mM MgCl$_2$, 10 mM 2-mercaptoethanol, 6.7 µM EDTA and 16.7 mM (NH$_4$)$_2$SO$_4$ which contains 200 µM αATP, αCTP, αTTP and αGTP for 30 minutes in order to fill in the BamHI cleavage end. The reaction mixture is subjected to phenol extraction and ethanol precipitation as described above. The resulting precipitates are subjected to a linking reaction with an Xho I linker in a molar ratio of 1:10 with T4 DNA ligase under the same conditions as described hereinbefore. After phenol extraction and ethanol precipitation, the resulting plasmid is treated with Xho I to give an HBsAg coding sequence (about 1.3 kb) having the Xho I cleavage terminus at both ends. The 1.3 kb fragment is recombined with the Xho I cleaved shuttle vector pAM 82 in a molar ratio of 5:1 by annealing and ligating with T4 DNA ligase. *E. coli* x1776 is transformed with the reaction mixture in the same manner as described in the step 3 (i) to give plasmid DNA.

Into the plasmid thus obtained is inserted the HBsAg coding sequence in the correct orientation downstream of the phosphatase promoter of the vector pAM 82. The obtained plasmid is designated pAS 101.

(3) Preparation of transformed yeast

The starting yeast is *Saccharomyces cerevisiae* AH22 [a, leu2, his4, can1 (Cir$^+$)], which has been deposited at the Fermentation Research Institute, Agency of Industrial Science and Technology, Japan according to the Budapest Treaty as "FERM BP-312". The starting yeast is inoculated in YPD medium (100 ml) consisting of 2% polypeptone, 1% yeast extract and 2% glucose. The mixture is incubated at 30°C overnight, and thereafter, the cells are collected by centrifugation. The cells thus collected are washed with sterilized water (20 ml), suspended in a solution (5 ml) of 1.2 M sorbitol and 100 μg/ml Zymolyase®-60,000 (manufactured by Seikagaku Kogyo K.K., Japan), and the suspension is allowed to stand at 30°C for 30 minutes to give spheroplasts. The spheroplasts thus prepared are washed with 1.2 M sorbitol solution three times, and then suspended in a solution (0.6 ml) of 2 M sorbitol, 10 mM $CaCl_2$ and 10 mM Tris-HCl (pH 7.5). The suspension thus prepared is divided into small test tubes in a volume of 60 μl. To the suspension is added the solution of the recombinant plasmid pAH 203 (30 μl) prepared in the above (3). After mixing well, 0.1 M $CaCl_2$ (3 μl) is added to a final concentration of 10 mM $CaCl_2$, and the mixture is allowed to stand at room temperature for 5 to 10 minutes. To the resulting mixture is added each 1 ml of a solution of 20% polyethylene glycol 4,000, 10 mM $CaCl_2$ and 10 mM Tris-HCl (pH 7.5), and the mixture is allowed to stand at room temperature for about 20 minutes. The resulting mixture (each 0.2 ml) is added to a medium (10 ml) consisting of 22% sorbitol, 2% glucose, 0.7% yeast nitrogen base amino acid, 2% YPD, 20 μg/ml histidine and 3% agar, which is kept at a constant temperature of 45°C. After gentle mixing, the mixture is added in a layer onto a plate of minimal medium containing 1.2 M sorbitol which is previously prepared and consists of 0.7% yeast nitrogen base amino acid, 2% glucose, 20 μg/ml histidine and 2% agar and is set thereon. The plate is incubated at 30°C to give colonies of a leucine-non-requiring yeaste. The colonies are incubated in a BurkHolder minimal medium supplemented with histidine (20 μg/ml) [cf. Tohe, A., et al., J. Bacteriol., *113*, 727—738, 1973] to give the desired transformed yeast: *Saccharomyces cerevisiae* pAH 203.

In the same manner as described above, except that the recombinant plasmid PAS 101 is used instead of the recombinant pAH 203, *Saccharomyces cerevisiae* pAS 101 is prepared.

(4) Production of HBsAg with the transformed yeast

Each colony of the transformed yeasts obtained in the above (4) is applied onto an agar plate of BurkHolder minimal medium supplemented with histidine (20 μg/ml) and incubated at 30°C to form a colony (in order to confirm the transformant requiring no leucine). The resulting cells are separated from the colony inoculated into BurkHolder minimal medium supplemented with histidine (20 μg/ml) and incubated at 30°C. After about 24 hours, the cells in exponential growth phase are collected by centrifugation, suspended in a minimal medium (10 ml) containing no phosphoric acid (which is prepared by replacing $KH_2PO_4$ in BurkHolder minimal medium with KCl, followed by supplementing with 20 μg/ml histidine) in a cell concentration of about $4 \times 10^6$ cells/ml. After an incubation at 30°C for about 24 hours, the cells are collected by centrifugation of the culture broth at 4,000 r.p.m. for 10 minutes. The cells thus separated are suspended in a solution (3 ml) of 1.2 M sorbitol, 50 mM phosphate buffer (pH 7.2), 14 mM 2-mercaptoethanol and 100 μg/ml Zymolyase®-60,000 (manufactured by Seikagaku Kogyo K.K., Japan), and the mixture is gently shaken at 30°C for 30 minutes to give spheroplasts. The spheroplasts are collected by centrifugation and are well suspended in a solution (1 ml) of 0.1% Tritone®X-100 and 50 mM phosphate buffer (pH 7.2), stirred vigorously and then centrifuged at 7,000 r.p.m. for 10 minutes, and the resulting supernatant is taken as the yeast-lysate solution.

The lysate solution (20 μl) obtained is tested with an HBs antigen RIA kit (manufactured by Abbott, U.S.A.) for HBsAg activity. The results are shown in Table 1.

TABLE 1

| Clone No. | Host | Plasmid | HBsAg Activity (cpm) |
|---|---|---|---|
| 1 | *S. cerevisiae* AH22 (FERM BP-312) | pAH 203 | 13,008 |
| 2 | " | pAS 101 | 11,200 |
| Reference | " | pAM 82* | 320 |

\* This vector contains no HBV or HBs coding sequence and is used as a negative reference. (The negative control of the RIA kit has an activity of 310 cpm, and the positive control thereof has that of 17,500 cpm).

6

**(5) Preparation of an HBcAg expression plasmid**

An HBcAg coding sequence is prepared by the following procedure.

Plasmid pHBV (3 µg) is digested with the restriction endonuclease Rsa I in the usual manner. The reaction mixture is subjected to phenol extraction and ethanol precipitation as described above. The resulting precipitates are subjected to a linking reaction with an Xho I linker in a molar ratio of 1:10 with T4 DNA ligase. After phenol extraction and ethanol precipitation, the resulting precipitates are subjected to Xho I digestion to give an HBc coding sequence (about 0.7 kb) having an Xho I cleavage terminus at both ends.

The fragment thus obtained is recombined with the Xho I cleaved shuttle vector pAM 82 in a molar ratio of 5:1 by annealing and ligating with T4 DNA ligase. The reaction mixture is used to transform *E. coli* x1776 in the same manner as described in (1) (ii) (B) to give a plasmid DNA. Into the plasmid thus obtained is inserted the HBcAg coding sequence in the correct orientation downstream of the phosphatase promoter of the vector pAM 82. The plasmid is designated pHC 301.

**(6) Preparation of transformed yeast**

The starting yeast is *Saccharomyces cerevisiae* AH22 [a, leu2, his4, can1 (Cir$^+$)], which has been deposited at the Fermentation Research Institute, Agency of Industrial Science and Technology, Japan according to the Budapest Treaty as "FERM BP-312". The starting yeast is inoculated in YPD medium (100 ml) consisting of 2% polypeptone, 1% yeast extract and 2% glucose. The mixture is incubated at 30°C overnight, and thereafter, the cells are collected by centrifugation. The cells thus collected are washed with sterilized water (20 ml), suspended in a solution (5 ml) of 1.2 M sorbitol and 100 µg/ml Zymolyase®-60,000 (manufactured by Seikagaku Kogyo K.K., Japan), and the suspension is allowed to stand at 30°C for 30 minutes to give spheroplasts. The spheroplasts thus prepared are washed with 1.2 M sorbitol solution three times, and then suspended in a solution (0.6 ml) of 2 M sorbitol, 10 mM CaCl$_2$ and 10 mM Tris-HCl (pH 7.5). The suspension thus prepared is divided into small test tubes in a volume of 60 µl. To the suspension is added the solution of the recombinant plasmid pAC 301 (30 µl) prepared in the above (5). After mixing well, 0.1 M CaCl$_2$ (3 µl) is added to a final concentration of 10 mM CaCl$_2$, and the mixture is allowed to stand at room temperature for 5 to 10 minutes. To the resulting mixture is added each 1 ml of a solution of 20% polyethylene glycol 4,000, 10 mM CaCl$_2$ and 10 mM Tris-HCl (pH 7.5), and the mixture is allowed to stand at room temperature for about 20 minutes. The resulting mixture (each 0.2 ml) is added to a medium (10 ml) consisting of 22% sorbitol, 2% glucose, 0.7% yeast nitrogen base amino acid, 2% YPD, 20 µg/ml histidine and 3% agar, which is kept at a constant temperature of 45°C. After gentle mixing, the mixture is added in a layer onto a plate of minimal medium containing 1.2 M sorbitol which is previously prepared and consists of 0.7% yeast nitrogen base amino acid, 2% glucose, 20 µg/ml histidine and 2% agar and is set thereon. The mixture is incubated at 30°C to give colonies of a leucine-non-requiring yeast. The colonies are incubated in a BurkHolder minimal medium supplemented with histidine (20 µg/ml) [cf. Tohe, A., et al., J. Bacteriol., *113*, 727—738, 1973] to give the desired transformed yeast: *Saccharomyces cerevisiae* pHC 301.

**(7) Production of HBcAg with the transformed yeast**

Each colony of the transformed yeasts obtained in the above (6) is applied onto an agar plate of BurkHolder minimal medium supplemented with histidine (20 µg/ml) and incubated at 30°C to form a colony (in order to confirm the transformant requiring no leucine). The resulting cells are separated from the colony, inoculated into BurkHolder minimal medium supplemented with histidine (20 µg/ml) and incubated at 30°C. After about 24 hours, the cells in the exponential growth phase are collected by centrifugation, suspended in a minimal medium (10 ml) containing no phosphoric acid (which is prepared by replacing KH$_2$PO$_4$ in BurkHolder minimal medium with KCl, followed by supplementing with 20 µg/ml histidine) in a cell concentration of about $4 \times 10^6$ cells/ml. After an incubation at 30°C for about 24 hours, the cells are collected by centrifugation of the culture broth at 4,000 r.p.m. for 10 minutes. The cells thus separated are suspended in a solution (3 ml) of 1.2 M sorbitol, 50 mM phosphate buffer (pH 7.2), 14 mM 2-mercaptoethanol and 100 µg/ml Zymolyase®-60,000 (manfuactured by Seikagaku Kogyo K.K., Japan), and the mixture is gently shaken at 30°C for 30 minutes to give spheroplasts. The spheroplasts are collected by centrifugation and are well suspended in a solution (1 ml) of 0.1% Tritone®X-100 and 50 mM phosphate buffer (pH 7.2), stirred vigorously and then centrifuged at 7,000 r.p.m. for 10 minutes, and the resulting supernatant is taken as the yeast-lysate solution.

The lysate solution (20 µl) obtained is tested with an HBc antigen RIA kit (manufactured by Abbott, U.S.A.) for the HBcAg activity. The results are shown in Table 2.

# EP 0 103 201 B1

## TABLE 2

| | Host | Plasmid | HBcAg Activity (cpm) |
|---|---|---|---|
| | *S. cerevisiae* AH22 (FERM BP-312) | pHC 301 | 4,989 |
| Reference | „ | pAM 82* | 16,913 |

\* This vector contains no HBV or HBs-coding sequence and is used as a negative reference. (The negative control of the RIA kit has an activity of 17,740 cpm, and the positive control thereof has that of 446 cpm).

## Claims

1. A shuttle vector comprising the following components:

(a) DNA sequences of the yeast *Saccharomyces cerevisiae* including ars 1, the 2 µ ori, and a marker gene for a transformed yeast permitting the synthesis of leucine, histidine, tryptophane, uracil or adenine by the transformant;

(b) a DNA sequence of the *E. coli* plasmid pBR 322 which includes a marker gene for a transformed *E. coli* encoding a resistance for ampicillin as well as the origin of replication; and

(c) the expression control region of the repressible acid phosphatase gene of yeast without the entire phosphatase structural gene and without a region in the range of from +1 (ATG) to −100 bp upstream of the structural gene.

2. The shuttle vector according to claim 1, wherein the region to be deleted usptream of the phosphatase structural gene is in the range of from +1 to −50 bp.

3. The shuttle vector according to claim 1, wherein the region to be deleted upstream of the phosphatase structural gene is in the range of from −1 to −33 bp.

4. The use of the shuttle vector according to any one of claims 1 to 3 in a process for the production of recombinant expression plasmids.

## Patentansprüche

1. Pendelvektor, umfassend die folgenden Bestandteile:

(a) DNA-Sequenzen aus der Hefe Saccharomyces cerevisiae, einschließlich ars 1, dem 2 µ ori, ein einer transformierten Hefe die Synthese von Leucin, Histidin, Tryptophan, Uracil oder Adenin durch die Transformante erlaubendes Markergen;

(b) eine DNA-Sequenz aus dem E. coli-Plasmid pBR322, die ein für ein transformiertes E. coli Ampicillinresistenz kodierendes Markergen sowie den Ursprung der Replikation umfaßt;

(c) den Expressionskontrollbereich des reprimierbaren Gens der sauren Phosphatase von Hefe ohne das gesamte Strukturgen der Phosphatase und ohne den Bereich von +1 (ATG) bis −100 bp stromaufwärts von dem Strukturgen.

2. Pendelvektor nach Anspruch 1, wobei der stromaufwärts von dem Strukturgen der Phosphatase zu deletierende Bereich von +1 bis −50 bp reicht.

3. Pendelvektor nach Anspruch 1, wobei der stromaufwärts von dem Strukturgen der Phosphatase zu deletierende Bereich von +1 bis −33 bp reicht.

4. Die Verwendung des Pendelvektors nach einem der Ansprüche 1 bis 3 in einem Verfahren zur Herstellung von rekombinanten Expressionsplasmiden.

## Revendications

1. Vecteur navette comprenant les composants suivants:

(a) des séquences d'ADN de la levure *Saccharomyces cerevisiae* comprenant ars 1, le 2 µ ori et un gène marqueur pour une levure transformée permettant la synthèse de la leucine, de l'histidine, du tryptophane, de l'uracile ou de l'adénine par le transformant;

(b) une séquence d'ADN du plasmide pBR322 d'*E. coli* qui a une taille d'environ 3,7 kb et qui comprend un gène marqueur pour un *E. coli* transformé codant pour une résistance à l'ampicilline ainsi que l'origine de réplication et

(c) la région de contrôle de l'expression du gène de la phosphatase acide répressible de levure sans la totalité du gène de structure de la phosphatase et sans une région dans l'intervalle de +1 (ATG) à −100 pb en amont du gène de structure.

2. Vecteur navette selon la revendication 1, dans lequel la région à supprimer en amont du gène de structure de la phosphatase est dans l'intervalle de +1 à −50 pb.

3. Vecteur navette selon la revendication 1, dans lequel la région à supprimer en amont du gène de structure de la phosphatase est dans l'intervalle de −1 à −33 pb.

4. Utilisation du vecteur navette selon l'une quelconque des revendications 1 à 3 dans un procédé pour la production de plasmides d'expression recombinants.

EP 0 103 201 B1

Fig. 1

1. Sal I
2. Bal 31
3. XhoI linker
   T4 DNA ligase.

pAT77

pAM82

Fig. 2

1

Fig. 3

Bst EII
     ↓    -170        -160        -150        -140        -130
              GTCACCTTACTTGGCAAGGCATATACCCATTTGGGATAAAGGGTAAACACTT

-120        -110        -100        -90        -80        -70        -60        -50
TGAATT(G)TCGAAAATGAAACGTATATAAGACGCTGATGTTTTGCTAAGTCGAGGTTAGTATGGCTTCATCTCTCA

-40        -30        -20        -10        +1                +82
TGAGAATAAGAACAACAACAAATAGAGCAAGCAAATTCGAGATTACCAATG..................GTCGAC
                                                    Sal I